(19) Europäisches Patentamt

European Patent Office

Office européen des brevets

(11) EP 0 575 967 B1

(12) **EUROPÄISCHE PATENTSCHRIFT**

(45) Veröffentlichungstag und Bekanntmachung des Hinweises auf die Patenterteilung:
**10.09.1997 Patentblatt 1997/37**

(51) Int. Cl.$^6$: **A61C 1/00**, A61C 1/18

(21) Anmeldenummer: **93109969.1**

(22) Anmeldetag: **22.06.1993**

(54) **Schlauchkennung**

Hose identification

Tuyau d'identification

(84) Benannte Vertragsstaaten:
**CH DE FR IT LI**

(30) Priorität: **23.06.1992 DE 4220522**

(43) Veröffentlichungstag der Anmeldung:
**29.12.1993 Patentblatt 1993/52**

(73) Patentinhaber:
**KALTENBACH & VOIGT GMBH & CO.**
**D-88400 Biberach (DE)**

(72) Erfinder:
• **Gmeinder, Hermann**
  **D-7950 Biberach/Mettenberg (DE)**

• **Baumann, Linus**
  **D-7955 Belamont (DE)**
• **Schmid, Gerhard**
  **D-7951 Mittelbiberach (DE)**

(74) Vertreter: **Schmidt-Evers, Jürgen, Dipl.-Ing. et al**
**Patentanwälte**
**Mitscherlich & Partner,**
**Postfach 33 06 09**
**80066 München (DE)**

(56) Entgegenhaltungen:
EP-A- 0 110 200          WO-A-91/00067
DE-A- 3 339 650          DE-A- 3 706 264
FR-A- 2 492 254          FR-A- 2 571 606
FR-A- 2 651 624          US-A- 4 401 949

**Beschreibung**

Die vorliegende Erfindung betrifft einen zahnärztlichen Behandlungsplatz, speziell eine Vorrichtung zum Erkennen der auf einen Schlauch aufgesteckten Instrumententeile.

Aus der EP 0 110 200 ist eine Schlauchkennungsvorrichtung bekannt. Eei dieser Vorrichtung ist ein zahnärztliches Behandlungsinstrument einem bestimmten Medien führenden Schlauch zugeordnet. Das geräteseitige Ende des Schlauchs ist mit Stiften versehen. Die geräteseitigen Anschlüsse weißen entsprechende die Stifte aufnehmende Buchsen auf. Durch die Kombination der Stifte (es können einige entfernt sein) wird dem Gerät signalisiert, welches Instrument an der betreffenden Gerätebuchse angeschlossen ist. Die Anzahl der Codierungsmöglichkeiten ist dabei direkt von der Anzahl der Stifte vorgegeben.

Nachteilig dabei ist, daß der Schlauch einem Verbraucher fest zugeordnet sein muß, wodurch eine Kaskadenbildung der am instrumentenseitigen Ende des Schlauchs aufgesteckten Teile nicht identifizierbar ist.

Aus der WO-A-91/00067 ist eine Vorrichtung gemäß dem Oberbegriff des Anspruches 1 zur Kenntlichmachung verschiedener Verbraucher bekannt.

Die Aufgabe der vorliegenden Erfindung besteht darin, eine Vorrichtung anzugeben, die mit möglichst geringem Aufwand eine hohe Anzahl von Codierungsmöglichkeiten erlaubt. Desweiteren soll es möglich sein, einem Schlauch verschiedene Instrumente zuordnen zu können. Das bedeutet, daß an das instrumentenseitige Ende eines Schlauchs verschiedene Instrumente angeschlossen werden können und daß dem Gerät über den Schlauch signalisiert wird, welches Instrument angeschlossen ist. Diese Aufgabe wird durch die kennzeichnenden Merkmale des Anspruchs 1 gelöst.

Weitere vorteilhafte Ausgestaltungen der Erfindung sind in den Unteransprüchen angegeben.

Die Erfindung soll anhand der folgenden Figuren beispielhaft erläutert werden. Es zeigen

**Figur 1** exemplarisch die Anordnung mehrerer jeweils einem Behandlungsplatz-Element zugeordneten Leistungs-Moduln und Steuerschaltungen;
**Figur 2** exemplarisch den Aufbau eines Behandlungsplatz-Elements;
**Figur 3** eine Anordung zur Instrumentenkennung;
**Figur 4** die verschiedenen Möglichkeiten, ein Behandlungsinstrument zu konfigurieren;
**Figur 5** die Ausführung der Kontaktgabe von zwei Instrumententeilen für die Instrumentenkennung
**Figur 6** exemplarisch die Anordnung von Steuerschaltungen und einer externen Speicher-Einheit; und
**Figur 7** exemplarisch den Aufbau einer solchen externen Speicher-Einheit; und
**Figur 8** die Anordnung zur Regelung der Helligkeit von Anzeigeeinheiten.

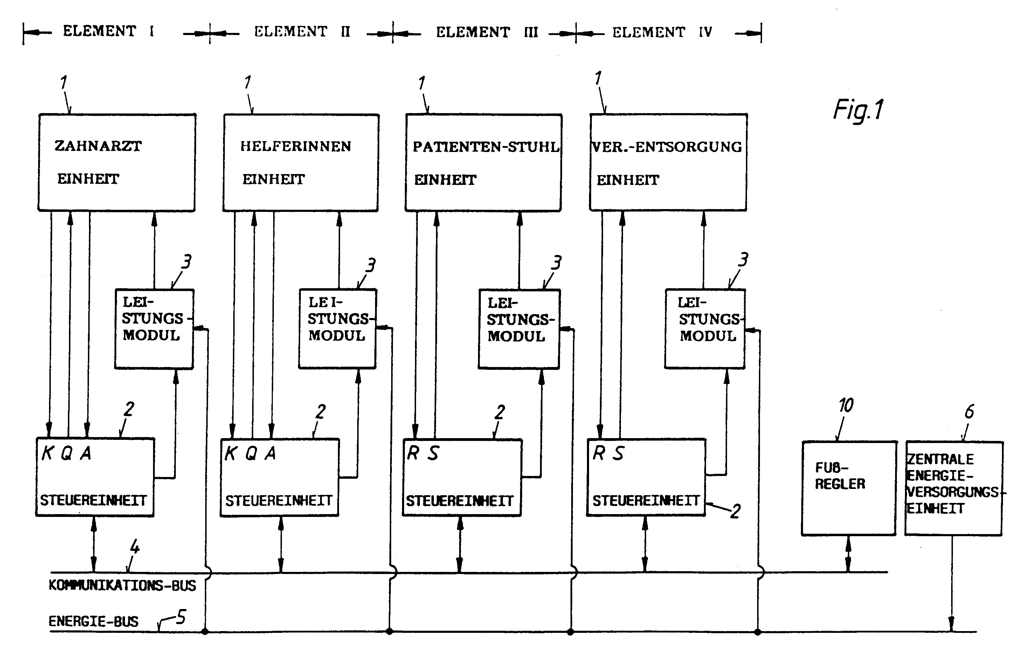

Fig. 1 zeigt den Aufbau mehrerer Behandlungsplatz-Elemente, wobei in der Zeichnung jeweils zwei Elemente funktionell gleichartig sind. Selbstverständlich kann ein Behandlungsplatz auch mehr als die gezeigten vier Behandlungsplatz-Elemente aufweisen. Die Zuordnung einzelner Verbraucher zu einem Behandlungsplatz-Element erfolgt normalerweise nach der räumlichen Plazierung bzw. der funktionellen Zusammengehörigkeit der Verbraucher. Beispielsweise werden die vom Arzt zu bedienenden Instrumente einem Arzt-Element (Element I) und die von der Arzthelferin zu bedienenden Instrumente einem Helferinnen-Element (Element II) zugeordnet. Die zur Ausführung der Patientenstuhl-Funktionen notwendigen Einstellungen übernimmt ein Patientenstuhl-Element (Element III) und für die Ver- und Entsorgung des Speibeckens kommt beispielsweise ein Ver- und Entsorge-Element (Element IV) zum Einsatz.

Jeder Verbraucher-Einheit 1 ist ein Leistungs-Modul 3 und eine Steuereinheit 2 zugeordnet. Jeder Leistungs-Modul stellt die den zu versorgenden Verbrauchern passenden Medien in Art und Menge zur Verfügung, wobei die Steuereinheit 2 dem Leistungs-Modul 3 meldet, welcher Verbraucher eingesetzt wird. Alle Steuereinheiten 2 können über einen gemeinsamen Kommunikations-Bus 4 miteinander Daten austauschen. Mit einem daran angeschlossenen Fußregler 10 lassen sich auch ausgesuchte Funktionen verschiedener Elemente steuern.

Die Leistungs-Moduln 3 erhalten die notwendigen "Roh"-Medien über einen Energie-Bus 5, der widerum an eine zentrale Energie-Versorgungs-Einheit 6 angeschlossen ist.

Eine detaillierte Betrachtung eines Elements soll anhand des Zahnarzt-Element (Element I) exemplarisch erfolgen. Die übrigen Elemente sind von ihrem Aufbau und ihrer Funktion im wesentlichen vergleichbar. Deutliche Unterschiede werden jedoch erwähnt.

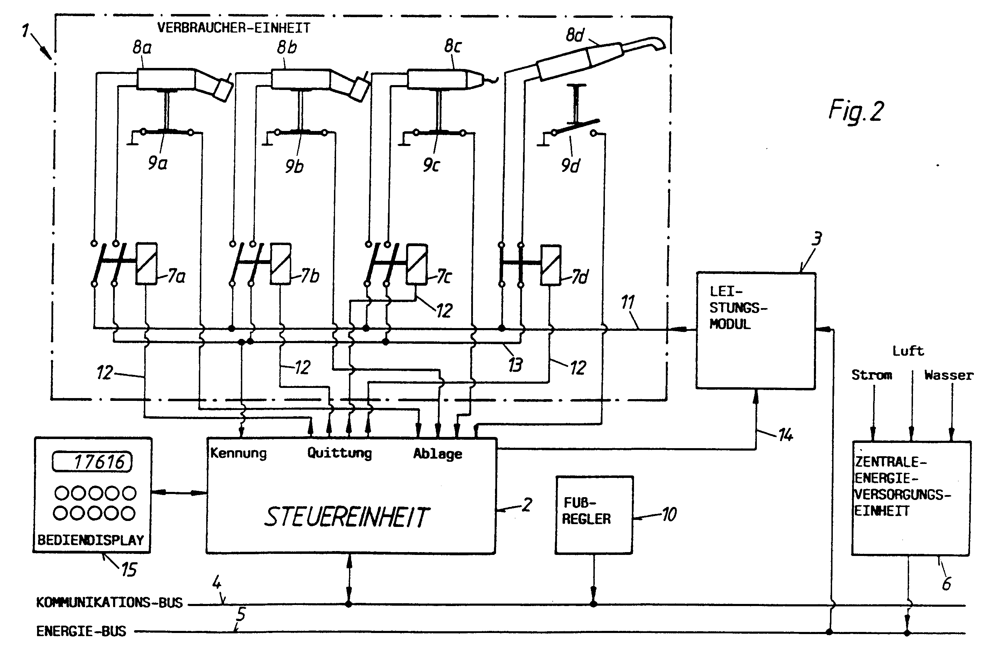

Fig.2 zeigt den detaillierten Aufbau des Arzt-Elements (Element I). Wie in Fig. 1 sind die Steuereinheit 2, die Zahnarzt-Einheit 1 und der Leistungs-Modul 3 zu erkennen. Zusätzlich sind noch ein Fußregler 10, die zentrale Energie-Versorgungs-Einheit 6 und ein Bediendisplay 15 dargestellt.

Die Zahnarzt-Einheit 1 umfaßt 4 Instrumente 8a...d, wie beispielsweise einen elektrisch angetriebenen Bohrer 8a, einen Turbinen-Bohrer 8b, ein Zahnsteinentfernungsgerät 8c und ein Aushärtelicht 8d. Die vier Instrumnte sind über ein busartig aufgebautes Versorgungsleitungssystem 11 mit dem Leistungs-Modul 3 verbunden, wobei steuerbare Schalter 7a...d das gewünschte Instrument zuschalten. Diese Schalter 7a...d, bspw. mechanische oder Halbleiterschalter, bekommen von der Steuereinheit 2 jeweils den Öffne- bwz. Schließ-Befehl über an den Ausgängen "Quittung" der Steuereinheit 2 angeschlossene Steuerleitungen 12.

Das Ansteuern dieser Schalter 7 erfolgt nach Auswertung eines Aktivierungssignals von einem der Ablageschaltern 9a...d. Jedem Instrument ist ein solcher Ablageschalter zugeordnet, der beim Herausnehmen des Instruments aus der Ablage öffnet und auf diese Weise der Steuereinheit 2 das Aktivierungssignal übermittelt.

Die Ablageschalter 9a...d können beispielsweise in Form von Lichtschranken gesteuerten Schaltern ausgebildet sein, wobei die jeweils einem Instrument zugeordneten Lichtschranken detektieren, ob ein Instrument aus der Ablage genommen wurde oder noch am vorgesehenen Platz liegt.

Zur Erhöhung der Bedienersicherheit durchläuft das Quittungssignal, d.h. der Steuerbefehl für einen der Schalter 7a...d, eine in der Steuereinheit 2 vorhandene Blockierschaltung, die dafür sorgt, daß immer nur einer der Schalter 7a...d geschlossen ist. Auf diese Art und Weise kann man verhindern, daß beim Auswechseln eines Bohrkopfs durch die Arzthelferin dieser in Betrieb geht, während der Zahnarzt mit einem anderen Instrument behandelt. Die Schaltung ist so ausgelegt, daß das zuerst aus der Ablage entnommene Instrument die anderen Instrumente blockiert.

Nimmt beispielsweise der Zahnarzt das Aushärtelicht 8d, wie in Fig. 2 gezeigt, aus der Ablage, so gibt der zughörige Ablageschalter 9d durch sein Öffnen ein Signal an den Ablage-Eingang der Steuereinheit 2. Diese quittiert das Signal über eine entsprechende Steuerleitung 12 mit dem Schließen des Schalters 7d. Die übrigen Schalter 7a...c sind daraufhin blockiert. Mit dem Schließen des Schalters 7d erhält das Aushärtelicht 8d die entsprechenden Versorgungsmedien, in diesem Fall nur elektrische Energie, in der passenden Menge von dem Leistungs-Modul 3. Die übrigen Verbraucher 8a...c sind von dem Versorgungsleitungssystem 11 getrennt.

Das Leistungs-Modul 3 erhält die Information über die Art und Menge der zur Verfügung zu stellenden Versorgungsmedien über eine Steuerleitung 14 von der Steuereinheit 2.

Das Schließen des Schalters 7d bewirkt außerdem, daß ein Kennungssignal über die Kennungsleitung 13 am Kennungs-Eingang der Steuereinheit 2 anliegt und dort ausgewertet wird.

Natürlich ist der Zahnarzt auch in der Lage, die Kennungseinrichtung 16 passiv zu schalten und über ein Bediendisplay 15 alle notwendigen Werte per Hand einzugeben und über das Display zu kontrollieren.

Funktionsbefehle, die über den Fußregler 10 ausführbar sind, werden über den Kommunikationsbus 4 an die jeweils zuständige Steuereinheit 2 geführt.

Das besondere an der erfindungsgemäßen Anordnung liegt darin, daß für alle vier Verbraucher 8 nur ein Leistungs-Modul 3 vorgesehen ist, daß die von der zentralen Energie-Versorgungs-Einheit gelieferten Versorgungsmedien Strom, Luft und Wasser in passender Art und Menge den, ausgewählten Verbraucher zuführt.

Wie bereits erwähnt, erhält die Steuereinheit 2 von dem in Benutzung befindlichen Instrument 8d ein Kennungssignal. Dieses Signal wird von einer Kennungseinrichtung 16 ausgewertet. Dabei gibt der Signalwert, beispielsweise ein Spannungswert, Aufschluß über die Art und Beschaffenheit des ausgewählten Instruments 8d. Eine differenzierte Aussage über die angeschlossenen Bestandteile des Instruments, beispielsweise Schlauchtyp und Getriebeaufsatz, sind dadurch möglich.

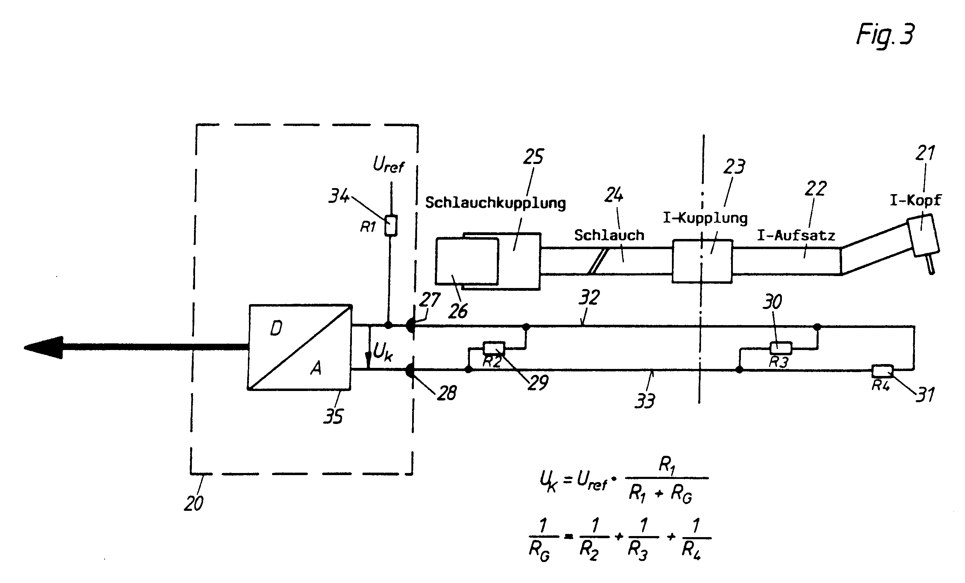

Fig.3 zeigt einen möglichen Aufbau einer solchen Instrumentenkennung.

Das zu erkennende Behandlungsinstrument kann beispielsweise einen Instrumentenkopf 21, einen Instrumentenaufsatz 22, eine Instrumentenkupplung 23, einen daran angeschlossenen Schlauch 24, der wiederum mit Hilfe eine Schlauchkupplung 25 und einem Gegenkupplungsglied 26 an ein Grundgerät angeschlossen ist, aufweisen.

Der zur Anerkennung notwendige elektrische Aufbau ist darunter abgebildet.

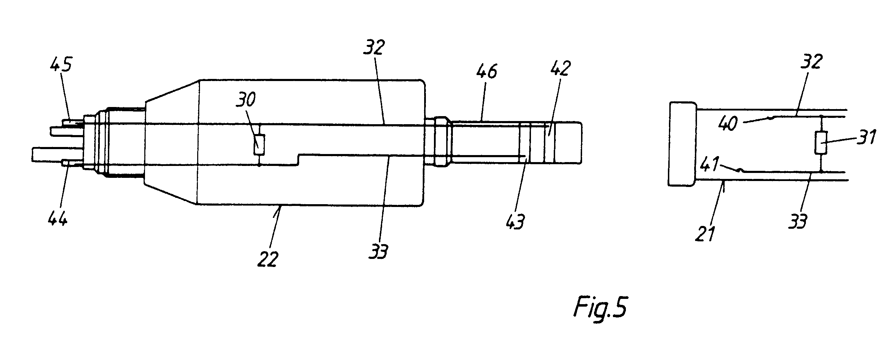

Zwei geräteseitige elektrische Kontakte 27, 28 werden durch ein Leitungspaar 32, 33, das sich von der Schlauchkupplung 25, durch den Schlauch 24, die Instrumentenkupplung 23, den Instrumentenaufsatz 22 bis zum Instrumentenkopf 21 erstreckt. Die elektrischen Verbindungen zwischen einzelnen Bestandteilen des Instruments werden entweder mit üblichen Steckkontakten oder mit Ringkontakten, die anhand von Fig. 5 erläutert werden, hergestellt. Jedes zu erkennende Instrumententeil weist einen Widerstand 29, 30, 31 auf, der das Leitungspaar 32, 33 verbindet. Demnach liegt an den Eingängen 27 und 28 eine Parallelschaltung aus drei Widerstanden.

Zur Erkennung des Instruments wird an einen Eingang 27 über einen Referrenzwiderstand eine Referrenzspannung angelegt. Daraufhin liegt zwischen den Eingängen 27 und 28 eine Spannung $U_K$, die vom Gesamtwiderstand der Widerstände 29, 30, 31 abhängt. Der gesamte Widerstand berechnet sich bekanntlich nach der Formel

$$\frac{1}{R_G} = \frac{1}{R_2} + \frac{1}{R_3} + \frac{1}{R_4}$$

Wählt man für jedes Instrumententeil einen charakteristischen Widerstand, läßt sich anhand der Spannung $U_K$, die proportional zum Gesamtwiderstand ist, das Instrument eindeutig identifizieren.

Die an den Eingängen 27 und 28 anliegende Spannung $U_K$ wird einer Meßeinrichtung, beispielsweise ein Analog/Digital-Wandler, zugeführt. Diese ermittelt den digitalen Wert der Spannung $U_K$ und liefert diesen an die Steuereinheit 2. Anhand des digitalisierten Meßwerts und mit Hilfe von gespeicherten Tabellen läßt sich genau sagen, aus welchen Teilen das Instrument zusammengesetzt ist. Basierend auf diesem Ergebnis, kann die Steuereinheit 2 dem Leistungs-Modul 3 Art und Menge der Versorgungsmedien vorgeben. Die Anzahl der codierbaren Instrumentenanordnungen ist im wesentlichen durch das Auflösungsvermögen des Analog/Digital-Wandlers begrenzt, obwohl lediglich zwei Kontakte pro Kupplung notwendig sind.

Statt einer Parallel-Schaltung der Widerstände 29, 30 und 31 könnte auch eine Serienschaltung eingesetzt werden.

Denkbar wäre natürlich auch, statt der Widerstände andere spannungs- bzw. stromverändernde Elemente zu verwenden, wie beispielsweise Kapazitäten oder Induktivitäten. Diese müßten dann mit einer Wechselspannung oder einzelnen Spannungsimpulsen beaufschlagt werden.

Entscheidend für die Erkennung ist nur, daß die an den Eingängen 27 und 28 gemessene Größe Aufschluß über die Eigenschaft des daran angeschlossenen Leitungssystems 32, 33 gibt, wobei jedes Instrumententeil 21, 22, 24 und 25 einen eigenen, eindeutigen Beitrag dazu liefert.

Vorteilhaft ist bei der angegebenen Anordnung vor allen Dingen, daß die Steuerschaltung 2 auch das Auswechseln des Instrumentenkopfs 21 oder des Instrumentenaufsatzes 22 erkennt und entsprechend reagieren kann. Die Steuerschaltung 2 ist somit in der Lage, schon beim Auswechseln eines Getriebeaufsatzes, die Betriebsparameter auf die neue Situation abzustimmen.

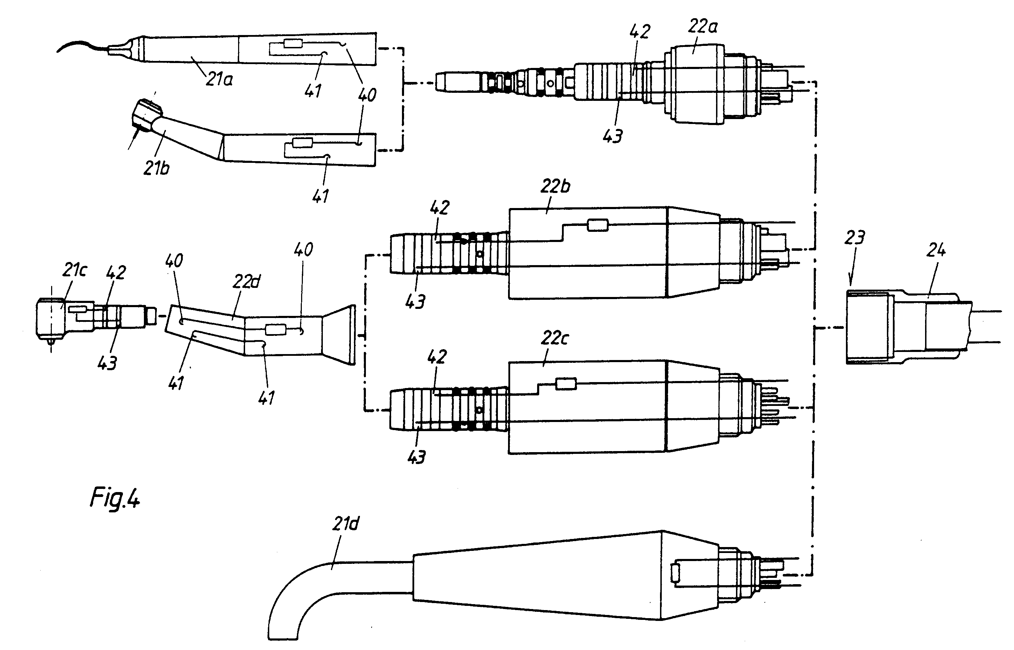

Fig. 4 zeigt einen kleinen Ausschnitt aus der Vielfalt der Anschlußmöglichkeiten von verschiedenen Instrumentenköpfen an einen als Universalschlauch ausgebildeten Schlauch 24. So sind beispielsweise das Zahnsteinentfernungsgerät 21a oder der Turbinenbohrer 21b über einen Instrumentenaufsatz 22a an den Schlauch 24 über eine Kupplung 23 anschließbar. Dies soll mit Hilfe der strichpunktierten Linie verdeutlicht werden. Desweiteren ist ein motorloser Instrumentenkopf 21c über ein Instrumtentenzwischenstück an einen Luftmotor 22b oder einen Elektromotor 22c an den Schlauch 24 anschließbar. Das Aushärtelicht 21d benötigt keine Zwischenstücke und ist direkt an den Schlauch 24 anschließbar.

Im Gegensatz zu Fig.3, sind in diesem Fall die zur Kennung notwendigen Widerstände in Serie geschaltet. Die elektrische Verbindung zwischen einzelnen Instrumententeilen erfolgt auf zweierlei Arten.

Normale Buchsen-Stecker-Kontakte übernehmen die Verbindung zwischen der Schlauchkupplung 23 und dem daran angeschlossenen Instrumententeil. Die elektrische Verbindung zwischen Instrumentenaufsatz und Instrumentenkopf erfolgt über federnde Kontakte 40, 41 und entsprechende ringförmige Gegenkontaktflächen 42, 43.

Fig. 5 verdeutlicht nochmals die benutzten Verbindungsarten.

Der Instrumentenkopf 21 wird zur Herstellung einer Verbindung auf das Endstück des Instrumentenaufsatzes 22 aufgesteckt. Beim Verbinden gleiten die federnd ausgebildeten Kontakte 40, 41 über die Außenfläche des entsprechenden Aufsatz-Endstücks. Im eingesteckten Zustand liegen die beiden Kontakte 40, 41 auf den ringförimig ausgebildeten Kontaktflächen 42 bzw. 43, wodurch eine elektrische Verbindung zwischen dem linken Leitungspaar 32, 33 hergestellt wird.

Die Qualität des elektrischen Kontakts hängt im wesentlichen vom Anpressdruck der federnden Kontakte 40, 41 ab, der durch die Form und das verwendete Material bestimmt ist.

Der Instrumenten-Aufsatz 22 verfügt an seiner anderen Seite über zwei Steckkontakte 44, 45, die mit dem Leitungspaar 32, 33 verbunden sind. Für die Steckkontakte 44, 45 sind an der Instrumentenkupplung 23 entsprechende Buchsenkontakte zur Herstellung einer elektrischen Verbindung vorgesehen.

Die zur Ausführung der beschriebenen Funktionen notwendigen Programmdaten sind normalerweise in einem Speicher der Steuereinheit 2 abgespeichert. Um eine einwandfreie Kommunikation der einzelnen Steuereinheiten 2 untereinander über den Kommunikationsbus zu ermöglichen, müssen die einzelnen Ausführungsprogramme aufeinander abgestimmt sein.

Die Programmdaten selbst werden normalerweise regelmäßig in Festwertspeichern (ROM's) gespeichert. Die Festwertspeicher selbst sitzen in dafür vorgesehenen Sockeln auf der Steuereinheit 2. Sollten Programmänderungen durchgeführt werden, müssen die entsprechenden Festwertspeicher ausgetauscht werden. Dies gilt meistens für die Festwertspeicher sämtlicher Behandlungsplatz-Elemente, da wie oben schon erwähnt, die Programme unterschiedlicher Elemente aufeinander abgestimmt sein müssen.

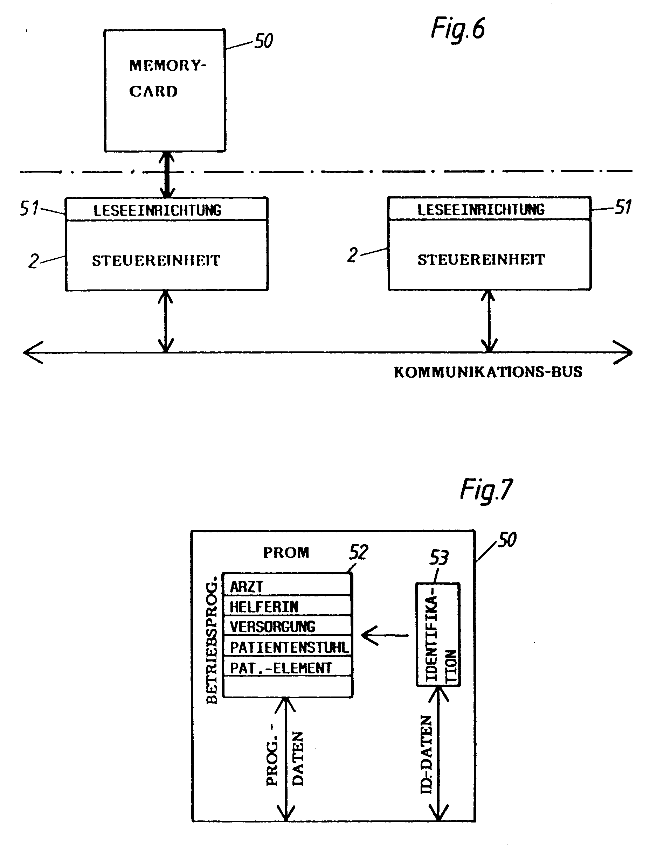

Fig. 6 zeigt eine Möglichkeit, wie das eben beschriebene Auswechseln vermieden werden kann. Dafür ist jeder Steuereinheit 2 eine Leseeinrichtung 51 zugeordnet, die von einem Datenträger 50, beispielsweise einer Magnetkarte oder ein E-PROM, die benötigten Programmdaten ausliest und im Speicher der Steuereinheit 2 ablegt.

Selbstverständlich ist auch denkbar, daß nur eine Steuerschaltung 2 eine solche Leseeinrichtung 51 umfaßt, wobei die für die anderen Steuerschaltungen 2 bestimmten Programmdaten über den Kommunikationsbus übertragen werden.

Weiterhin ist denkbar, daß die Leseeinrichtung 51 keiner Steuerschaltung 2 direkt zugeordnet ist, sondern über den Kommunikationsbus 4 jeder einzelnen Steuerschaltung 2 den zugehörigen Programmcode übermittelt.

Anhand der Figur 7 soll nochmals erläutert werden, wie sich ein solcher Programmaustausch vollzieht. Dabei wird von einer Speicher-Karte 50 ausgegangen, die als Datenträger ein PROM zur Speicherung der Betriebsprogramme enthält.

Beim Einführen der Speicher-Karte in die Leseeinrichtung, beispielsweise über einen Steckkontakt, überträgt zu allererst die Leseeinrichtung einen Identifikations-Code, der jede Steuerschaltung 2 eindeutig kennzeichnet und dort auch individuell einstellbar ist. Abhängig von diesem Identifikations-Code, der in einem Speicher 53 abelegt ist, wird ein entsprechender Bereich des Betriebsprogrammspeichers 52 adressiert und die darin abgespeicherten Daten von der Leseeinrichtung ausgelesen und in den entsprechenden Speicher der Steuerschaltung 2 eingeschrieben.

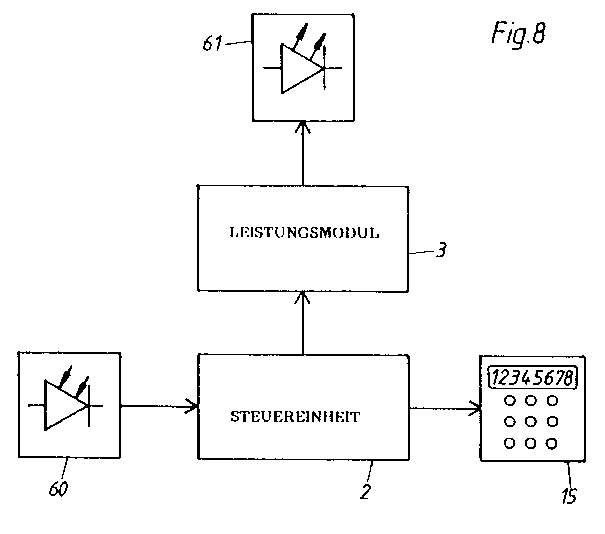

Wie bereits erwähnt, kann der Zahnarzt auf dem Display des Bedienerdisplays 15 oder anderen Anzeigeeinheiten (LED's) Informationen über verschiedene Funktionen ablesen. Die Ablesebarkeit des Displays bzw. der Leuchtdioden hängt sehr stark von der Umgebungshelligkeit im Behandlungsraum ab. Um auf diesen Parameter reagieren zu können, enthält die Steuereinheit 2 eine entsprechende Helligkeitssteuerung, die in Fig. 8 dargestellt ist. Dabei mißt ein lichtempfindliches Schaltelement 60, bspw. eine Photodiode, die Umgebungshelligkeit und überträgt einen entsprechenden Wert an die Steuereinheit 2. Diese stellt die LEDs 61, bzw. das Display des Bedienerdisplays 15 über eine Änderung der Versorgungsspannung ein. Die Regelung der Anzeigehelligkeit erfolgt proportional der Umgebungshelligkeit, d.h. je heller der Raum desto heller die Anzeige.

**Patentansprüche**

1. Vorrichtung zur Kenntlichmachung verschiedener Verbraucher (8), beispielsweise zahnärztlicher Instrumente, gegenüber einer zu einem zahnärztlichen Behandlungsplatz (1) gehörenden Energie-Versorgungseinheit (3), an die die Verbraucher jeweils über einen Versorgungsschlauch (24) anschließbar sind,

   mit an dem Versorgungsschlauch vorgesehenen Codierungsmitteln zur Identifizierung des dem Versorgungsschlauch zugeordneten Verbrauchers, wobei zu den Codierungsmitteln durch den Versorgungsschlauch bis zu dem Verbraucher hindurchgeführte elektrische Verbindungsleitungen (32, 33) gehören, die Teil eines Identifizierungsschaltkreises sind, und
   mit einer zu der Energie-Versorgungseinheit gehörenden Auswerteschaltung (20), welche mit den Codierungsmitteln verbindbar ist und die Energie-Versorgungseinheit (3) nach Auswertung der Codierung veranlaßt, dem Versorgungsschlauch (24) die zu dem daran angeschlossenen Verbraucher nach Art und Menge passenden Versorgungsmedien zuzuführen, wobei
   der Identifizierungsschaltkreis der Codierungsmittel in dem Verbraucher (8) vorgesehene Identifizierungs-Schaltelemente (29, 30, 31) einschließt, wobei sich die einzelnen Verbraucher durch voneinander abweichende Identifizierungs-Schaltelemente unterscheiden, dadurch gekennzeichnet, daß die Verbraucher (8) über Kaskaden an einen Verbindungsschlauch (24) anschließbar sind, wobei die Kaskaden durch einen Teil der den Identifizierungsschaltkreis bildenden Verbindungsleitungen (32, 33) miteinander verbunden sind und jede Kaskade mindestens ein dem Identifizierungsschaltkreis angegliedertes Identifizierungs-Schaltelement enthält.

2. Vorrichtung nach Anspruch 1,
   **dadurch gekennzeichnet,**
   daß die Identifzierungs-Schaltelemente (29,30,31) der einzelnen Kaskaden in Serie geschaltet sind.

3. Vorrichtung nach Anspruch 1,
   **dadurch gekennzeichnet,**
   daß die Identifizierungs-Schaltelemente (29,30,31) der einzelnen Kaskaden parallel geschaltet sind.

4. Vorrichtung nach einem der vorhergehenden Ansprüche,
   **dadurch gekennzeichnet,**
   daß die Identifzierungs-Schaltelemente (29,30,31) passive Schaltelemente sind.

5. Vorrichtung nach Anspruch 4,
   **dadurch gekennzeichnet,**
   daß die Identifizierungs-Schaltelemente (29,30,31) Widerstände und/oder Kapazitäten und/oder Induktivitäten sind.

6. Vorrichtung nach einem der vorhergehenden Ansprüche,
   **dadurch gekennzeichnet,**
   daß die Auswerteschaltung (20) einen Analog/Digital-Wandler (35) umfaßt, der eine zwischen den elektrischen Verbindungsleitungen (32,33) liegende Spannung mißt, wobei eine der Leitungen über einen Widerstand (34) an eine Referenz-Spannung ($U_{ref}$) gelegt ist.

7. Vorrichtung nach einem der vorhergehenden Ansprüche,
   **dadurch gekennzeichnet,**
   daß die elektrische Verbindung zwischen den einzelnen Kaskaden mittels federnd ausgebildeten Kontakten (40,41) erfolgt, die im zusammengesetzten Zustand zweier Kaskaden auf entsprechenden Gegenkontaktflächen (42,43) aufliegen.

8. Vorrichtung nach Anspruch 7,
   **dadurch gekennzeichnet,**
   daß die Gegenkontaktflächen (42,43) ringförmig augebildet sind.

**Claims**

1. A system for identifying different consuming devices (8), for example dental instruments, to an energy supply unit (3) belonging to a dental treatment station (1) to which the consuming devices can be connected in each case via a supply hose (24),

having coding means provided on the supply hose for identifying the consuming devices associated with said pply hose, wherein electrical connecting lines (32, 33) which pass through the supply hose up to the consuming device belong to the coding means, and are part of identification circuitry, and

an evaluation circuit (20) belonging to the energy supply unit that can be connected to the coding means, and which after evaluating the coding causes the energy supply unit (3) to supply to said supply hose (24) the appropriate type and amount of supply media for the consuming device connected thereto, wherein said identification circuitry of the coding means includes identification circuit elements (29, 30, 31) provided in the consuming device (8), the individual consuming devices differing by identification circuit elements that vary from one another,

**characterized in that**

the consuming devices (8) can be connected to a connecting hose (24) via cascades, wherein the cascades are connected to one another by some of the connecting lines (32, 33) forming the identification circuitry, and each cascade includes at least one identification circuit element associated with the identification circuitry.

2. An apparatus according to claim 1,
**characterized in that**
the identification circuit elements (29, 30, 31) of the individual cascades are connected in series.

3. An apparatus according to claim 1,
**characterized in that**
the identification circuit elements (29, 30, 31) of the individual cascades are connected in parallel.

4. An apparatus according to any one of the preceding claims,
**characterized in that**
the identification circuit elements (29, 30, 31) are passive circuit elements.

5. An apparatus according to claim 4,
**characterized in that**
the identification circuit elements (29, 30, 31) are resistors and/or capacitors and/or inductors.

6. An apparatus according to any one of the preceding claims,
**characterized in that**
the evaluation circuit (20) includes an analog/digital converter (35) that measures a voltage between the electrical connecting lines (32, 33), wherein a reference voltage ($U_{ref}$) is applied via a resistor (34) to one of the lines.

7. An apparatus according to any one of the preceding claims,
**characterized in that**
the electrical connection between the individual cascades is effected by means of resiliently formed contacts (40, 41) which, when two cascades are connected together, lie on corresponding counter contact surfaces (42, 43).

8. An apparatus according to claim 7,
**characterized in that**
said counter contact surfaces (42, 43) are annular.

**Revendications**

1. Dispositif pour l'identification d'appareils utilisateurs (8) différents, par exemple d'instruments de dentisterie, vis-à-vis d'un bloc (3) d'alimentation en énergie associé à un emplacement (1) de traitement dentaire auquel les appareils utilisateurs sont connectés chacun par un flexible (24) d'alimentation,

avec des moyens de codage prévus sur le flexible aux fins d'identifier l'appareil utilisateur associé au flexible d'alimentation, des lignes électriques (32, 33) disposées à l'intérieur du flexible menant à l'appareil utilisateur faisant partie des moyens de codage, lesquelles lignes électriques sont intégrées dans un circuit d'identification et
avec un circuit de traitement (20) appartenant au bloc d'alimentation en énergie qui peut être relié aux moyens de codage et qui, après traitement du code, donne l'ordre au bloc (3) d'alimentation en énergie d'envoyer au flexible d'alimentation (24) les types et quantités de fluides adaptées pour l'appareil utilisateur connecté,
le circuit d'identification des moyens de codage comprenant des éléments de circuit (29, 30, 31) d'identification prévus dans l'appareil utilisateur, les différents appareils utilisateurs étant différenciés par des éléments de circuit d'identification différents les uns des autres,
caractérisé par le fait que les appareils utilisateurs (8) peuvent être connectés par l'intermédiaire de cascades à un flexible d'alimentation (24), les cascades étant connectées les unes aux autres par une partie des lignes d'alimentation (32, 33) qui constituent le circuit d'identification et chaque cascade comportant au moins un élément de circuit d'identification associé au circuit d'identification.

2. Dispositif selon la revendication 1, caractérisé par le fait que les éléments de circuit (29, 30, 31) d'identification des différentes cascades sont branchés en série.

3. Dispositif selon la revendication 1, caractérisé par le fait que les éléments de circuit (29, 30, 31) d'identification des différentes cascades sont branchés en parallèle.

4. Dispositif selon l'une des revendications précédentes, caractérisé par le fait que les éléments de circuit (29, 30, 31) d'identification sont des éléments de circuit passifs.

5. Dispositif selon la revendication 4, caractérisé par le fait que les éléments de circuit (29, 30, 31) d'identification sont des résistances et/ou des capacités et/ou des inductances.

6. Dispositif selon l'une des revendications précédentes, caractérisé par le fait que le circuit de traitement (20) comporte un convertisseur analogique / numérique (35) qui mesure une tension entre les lignes (32, 33) électriques de liaison, l'une des lignes étant connectée à une tension de référence ($U_{ref}$) par l'intermédiaire d'une résistance (34).

7. Dispositif selon l'une des revendications précédentes, caractérisé par le fait que la liaison électrique entre les différentes cascades est réalisée à l'aide de contacts (40, 41) élastiques qui, lorsque deux cascades sont combinées, sont en appui sur des surfaces de contact (42, 43) conjuguées.

8. Dispositif selon la revendication 7, caractérisé par le fait que les surfaces de contact (42, 43) conjuguées sont conformées en anneaux.

Fig.1

Fig.2

VERBRAUCHER-EINHEIT

LEI-STUNGS-MODUL — 3

ZENTRALE-ENERGIE-VERSORGUNGS-EINHEIT — 6

Luft — Wasser — Strom

FUß-REGLER — 10

STEUEREINHEIT — 2

Kennung — Quittung — Ablage

17616 — BEDIENDISPLAY — 15

KOMMUNIKATIONS-BUS — 4

ENERGIE-BUS — 5

8a 8b 8c 8d — 9a 9b 9c 9d — 7a 7b 7c 7d — 11 — 12 — 13 — 14 — 1

9

Fig. 3

Fig.4

Fig.5

*Fig.6*

MEMORY-CARD —50

51— LESEEINRICHTUNG

2— STEUEREINHEIT

LESEEINRICHTUNG —51

2— STEUEREINHEIT

KOMMUNIKATIONS-BUS

*Fig.7*

PROM 52    53    50

BETRIEBSPROG.

ARZT
HELFERIN
VERSORGUNG
PATIENTENSTUHL
PAT.-ELEMENT

IDENTIFIKA-TION

PROG.-DATEN

ID-DATEN

Fig.8